# EUROPÄISCHE PATENTSCHRIFT

(11) **EP 3 616 624 B1**
(45) Veröffentlichungstag und Bekanntmachung des Hinweises auf die Patenterteilung: **28.04.2021**
(21) Anmeldenummer: 18191078.7
(22) Anmeldetag: 28.08.2018
(51) Int. Cl.: A61B 6/12, A61B 6/00

(54) **VERFAHREN ZUM BETRIEB EINER RÖNTGENEINRICHTUNG, RÖNTGENEINRICHTUNG, COMPUTERPROGRAMM UND ELEKTRONISCH LESBARER DATENTRÄGER**
METHOD FOR OPERATING AN X-RAY DEVICE, X-RAY DEVICE, COMPUTER PROGRAM AND ELECTRONICALLY READABLE DATA CARRIER
PROCÉDÉ DE FONCTIONNEMENT D'UN DISPOSITIF À RAYONS X, DISPOSITIF À RAYONS X, PROGRAMME INFORMATIQUE ET SUPPORT D'INFORMATIONS LISIBLE ÉLECTRONIQUEMENT

(43) Veröffentlichungstag der Anmeldung: 04.03.2020
(73) Patentinhaber: Siemens Healthcare GmbH, 91052 Erlangen (DE)
(72) Erfinder: SUTTER, Sven-Martin, 91074 Herzogenaurach (DE)

(56) Entgegenhaltungen:
- WO-A1-2017/106177
- DE-A1-102009 015 830
- US-A1- 2015 139 395
- US-A1- 2015 374 323
- US-A1- 2017 281 114

## Beschreibung

Die Erfindung betrifft ein Verfahren zum Betrieb einer Röntgeneinrichtung zur Aufnahme einer Bildserie zur insbesondere fluoroskopischen Überwachung eines Aufnahmebereichs eines Patienten, wobei Durchleuchtungsbilder mit einer Aufnahmerate und einer Aufnahmedosis erfasst und mit einer Bildrate einem Benutzer an einer Anzeigeeinrichtung dargestellt werden. Daneben betrifft die Erfindung eine Röntgeneinrichtung, ein Computerprogramm und einen elektronisch lesbaren Datenträger.

Die Röntgenbildgebung wird im Stand der Technik häufig zur Überwachung dynamischer Vorgänge in einem Aufnahmebereich innerhalb eines Patienten eingesetzt. Beispiele für derartige dynamische Vorgänge sind sogenannte Roadmap-Prozeduren, bei denen ein medizinisches Instrument, beispielsweise ein Katheter oder ein Führungsdraht, innerhalb eines Patienten zu einer Zielposition derart bewegt werden soll, dass möglichst geringe Beeinträchtigung des Patienten auftritt. Ein anderes Beispiel sind Bildgebungsvorgänge unter Verwendung von Kontrastmittel, bei denen beispielsweise die Kontrastmittelanflutung durch Durchleuchtungsbilder überwacht werden soll. Bei solchen Überwachungsvorgängen werden mit einer bestimmten Aufnahmerate Durchleuchtungsbilder des Patienten aufgenommen und unmittelbar bzw. nach einer bestimmten Nachbearbeitung dargestellt, das bedeutet, die Darstellung der Durchleuchtungsbilder auf einer Anzeigeeinrichtung schließt eine vorherige Bearbeitung der Durchleuchtungsbilder nicht zwangsläufig aus. Bekannt ist es beispielsweise, bestimmte Merkmale, beispielsweise das nachzuverfolgende medizinische Instrument, hervorzuheben und dergleichen. Während die bekannteste Aufnahmetechnik, bei der eine Bildserie zur Überwachung eines Aufnahmebereichs eines Patienten aufgenommen wird, die Fluoroskopie ist, sind auch andere Einsätze denkbar, beispielsweise bei der Aufnahme von Bildserien digitaler Radiographien.

Bei der Aufnahme einer Bildserie von Durchleuchtungsbildern zur Überwachung wird der Patient quasi kontinuierlich der Röntgenstrahlung ausgesetzt, so dass die Dosisreduktion für den Patienten ein wichtiges Thema bei solchen Überwachungsvorgängen, insbesondere der Fluoroskopie, ist. In diesem Kontext wurden bereits verschiedene Möglichkeiten vorgeschlagen, um die Dosisbelastung für einen Patienten zu reduzieren bzw. möglich gering zu halten. Zunächst werden derartige zur Überwachung eingesetzte Durchleuchtungsbilder meist mit äußerst geringer Aufnahmedosis erzeugt, da die relevanten, zu beobachtenden Merkmale, beispielsweise Kontrastmittel und/oder medizinische Instrumente, auch bei niedrigen Dosen hinreichend deutlich abgebildet werden. Um zusätzlich eine hinreichend genaue Darstellung der Anatomie zu erhalten, wurde vorgeschlagen, in der Darstellung eine Überlagerung von Durchleuchtungsbildern bzw. daraus abgeleiteten Anzeigebildern mit die Anatomie des Patienten deutlich zeigenden, beispielsweise präinterventionellen, Bilddaten vorzunehmen. Vorgeschlagen wurde ferner, das Strahlenfeld nur auf den unbedingt notwendigen Bereich zu kollimieren

In der Offenlegungsschrift DE 10 2009 015 830 wird vorgeschlagen, die Aufnahmeparameter, beispielsweise die Dosis, basierend auf einem ermittelten Vergleichswert anzupassen, mithilfe dessen die zwei zuletzt aufgenommenen Bilder im Hinblick auf die Lokalisierung eines Instruments beurteilt werden können.

In einem weiteren, ebenso in Stand der Technik bekannten Ansatz wurde vorgeschlagen, die Aufnahmerate der Durchleuchtungsbilder dann zu reduzieren, wenn keine oder eine nur geringe Veränderung von Durchleuchtungsbild zu Durchleuchtungsbild auftritt. Hierzu kann beispielsweise ein Maß für die Bildinhaltsveränderung zwischen den beiden aktuellsten Durchleuchtungsbildern ermittelt und mit einer Änderungsschwelle verglichen werden, um beispielsweise von einer Grundaufnahmerate von 30 fps auf eine Aufnahmerate von 10 fps oder weniger zurückzuschalten. Treten dann wieder stärkere Bildinhaltsveränderungen zwischen den aktuellsten Durchleuchtungsbildern auf, kann wieder auf die schnellere Aufnahmerate zurückgeschaltet werden, also im Beispiel die Grundaufnahmerate. Durch das Umschalten zwischen unterschiedlichen Aufnahmeraten verändert sich auch die Bildrate, mit der die Bilddaten der Durchleuchtungsbilder dargestellt werden.

Dieser Ansatz hat den Nachteil, dass auf plötzliche, schnell auftretende Veränderungen im Bildinhalt gegebenenfalls nicht oder auch nicht schnell genug reagiert werden kann, so dass dem Benutzer wesentliche Informationen entgehen können oder diese nicht mit der hinreichenden Qualität erkennbar sind.

Der Erfindung liegt daher die Aufgabe zu Grunde, eine Möglichkeit zur Dosisreduktion für den Patienten bei der Aufnahme einer Bildserie zur Überwachung anzugeben, die dennoch eine schnelle Reaktion auf auftretende stärkere Bildinhaltsveränderungen ermöglicht.

Zur Lösung dieser Aufgabe ist bei einem Verfahren der eingangs genannten Art erfindungsgemäß vorgesehen, dass die Aufnahmedosis in Abhängigkeit einer Bildinhaltsveränderung zwischen aktuellen, aufeinanderfolgenden Durchleuchtungsbildern automatisch gewählt wird. Insbesondere kann bei geringeren Bildinhaltsveränderungen eine niedrigere Aufnahmedosis gewählt werden als bei stärkeren Bildinhaltsveränderungen.

Um möglichst schnell auf plötzliche Bildinhaltsveränderungen stärkerer Art reagieren zu können, insbesondere mit einer Erhöhung der auf der Anzeigeeinrichtung dargestellten Bildrate, wird vorgeschlagen, bei geringgradigen Änderungen der Bildeinhalte nicht die Aufnahmerate selbst zu reduzieren, sondern die Aufnahmedosis dynamisch anzupassen, insbesondere die Aufnahmedosis pro Durchleuchtungsbild derart zu reduzieren, dass derartige Bildinhaltsveränderungen an relevanten Bildinhalten zwar noch äußerst schnell festgestellt werden können, gegebenenfalls jedoch die Qualität der Durchleuchtungsbilder für sich deutlich abnimmt.

In diesem Kontext sieht die vorliegenden Erfindung vor, dass bei einer Reduzierung der Aufnahmedosis aus jeweils mehreren aufeinanderfolgend aufgenommenen Durchleuchtungsbildern ein Anzeigebild, das einer maximal verwendbaren Grunddosis entspricht, ermittelt und an der Anzeigeeinrichtung ausgegeben wird. Dabei kann konkret vorgesehen sein, dass die Durchleuchtungsbilder durch Addition zu dem Anzeigebild kombiniert werden. Das bedeutet, dass insbesondere auch die - dem Bediener von den bekannten Vorgehensweisen gewohnte - Reduzierung der Bildrate, zumindest was die Anzeige auf der Anzeigeeinrichtung angeht, dennoch stattfinden kann. Hierzu kann vorgesehen sein, dass bei einer Ermittlung von Anzeigebildern die Bildrate reduziert wird, insbesondere um einen Faktor entsprechend der Anzahl der zusammengefassten Durchleuchtungsbilder bei disjunkten Gruppen zusammenzufassender Durchleuchtungsbilder. Mit anderen Worten kann die Aufnahmedosis zweckmäßiger Weise so reduziert werden, dass mehrere Durchleuchtungsbilder zusammengefasst, insbesondere addiert werden müssen, bevor diese wieder in hinreichender Qualität an der Anzeigeeinrichtung dargestellt werden können. So entsteht für den Bediener der ihm bekannte Bildeindruck, zusätzlich aber auch bevorzugt der ihm bekannte Bildratenwechsel. Hierbei werden letztlich immer Blöcke/Gruppen von aufeinanderfolgend aufgenommenen Durchleuchtungsbildern, insbesondere durch Addition, zu einem Anzeigebild zusammengefasst, so dass jeweils nach der Aufnahme einer Anzahl zusammenzufassender Durchleuchtungsbilder ein neues Anzeigebild ausgegeben werden kann, so dass sich die Bildrate um einen Faktor, der der Anzahl zusammenzufassender Durchleuchtungsbilder entspricht, reduziert.

Trotz dieser Zusammenfassung von Durchleuchtungsbildern zu einem Anzeigebild analysiert die Steuereinrichtung der Röntgeneinrichtung die mit der erniedrigten Aufnahmedosis aufgenommen Durchleuchtungsbilder einzeln, was Änderungen im Bildinhalt angeht, und passt die Aufnahmedosis, insbesondere zur erneuten Erhöhung auf die Grunddosis, gegebenenfalls wieder an, und zwar auf die Grunddosis, sobald hinreichend starke Bildinhaltsveränderungen auftreten. Zusammenfassend wird mithin vorgeschlagen, die Aufnahmedosis pro Durchleuchtungsbild entsprechend der dynamischen Bildinhaltsveränderung von Durchleuchtungsbild zu Durchleuchtungsbild bei Sequenzaufnahmen anzupassen und insbesondere auch die Bilddarstellungsfrequenz, also die Bildrate, an der Anzeigeeinrichtung entsprechend zu wählen. So kann die Steuereinrichtung der Röntgeneinrichtung schnell auf dynamische Änderungen der Bildinhalte reagieren, beispielsweise nach Injektion eines Kontrastmittelbolus und/oder bei einer schnelleren Bewegung eines medizinischen Instruments im Aufnahmebereich.

In einer besonders bevorzugten Weiterbildung der Erfindung kann vorgesehen sein, dass die Wahl der Aufnahmedosis ferner abhängig von einem Gradienten der Bildinhaltsveränderung über mehr als zwei aufeinanderfolgend aufgenommene, aktuelle Durchleuchtungsbilder erfolgt. Damit wird letztlich eine Art Geschwindigkeit der Bildinhaltsveränderung mit in die Betrachtungen einbezogen. Dabei kann insbesondere unter Berücksichtigung des Bildinhaltsveränderungsgradienten eine zukünftige Mindestbildinhaltsveränderung prädiziert werden und die neue Aufnahmedosis in deren Anhängigkeit gewählt werden.

Hierfür kann beispielsweise ein Prädiktionsalgorithmus verwendet werden, der insbesondere anwendungsbezogen gewählt sein kann, sich also beispielsweise an typischen Ausbreitungsgeschwindigkeiten von Kontrastmittel, das in den Aufnahmebereich eindringt, orientieren kann und/oder übliche Arbeitsgeschwindigkeiten mit medizinischen Instrumenten, insbesondere an verschiedenen Stellen des Workflows, beurteilen kann. Dabei können auch Techniken des Maschinenlernens, bzw. allgemein der künstlichen Intelligenz, herangezogen werden.

Dabei sein an dieser Stelle darauf hingewiesen, dass es auch grundsätzlich denkbar ist, Bildinhaltsveränderungen bzw. Bildinhaltsveränderungsintervallen entsprechende Aufnahmedosen, insbesondere anwendungsbezogen, zuzuordnen, beispielsweise in einer Look-Up-Tabelle und/oder durch einen mathematischen Zusammenhang.

Besonders zweckmäßig ist es im Rahmen der vorliegenden Erfindung jedoch, wenn die Veränderung der Aufnahmedosis in diskreten Stufen abhängig von der Überschreitung oder Unterschreitung von wenigstens einer Änderungsschwelle durch die Bildinhaltsveränderung erfolgt. Diese Stufen können dann insbesondere so gewählt werden, dass durch Zusammenfassen, insbesondere addieren, einer ganzzahligen Anzahl von aufeinanderfolgend aufgenommenen Durchleuchtungsbildern der Eindruck entsteht, das resultierende Anzeigebild sei mit der Grunddosis aufgenommen worden, sodass - bis auf gegebenenfalls leicht steigendes Rauschen - der Bildeindruck der Grunddosis für den Benutzer auf möglichst einfache Weise erhalten bleibt, insbesondere unter Reduzierung der Bildrate an der Anzeigeeinrichtung. Es können also gezielt Stufen der Aufnahmedosis gewählt werden, so dass durch Zusammenfassen, insbesondere durch Addition, der aufeinanderfolgenden Durchleuchtungsbilder einer Gruppe wieder der Eindruck einer Aufnahme mit der Grunddosis entsteht. Dabei ist es insbesondere denkbar, die Aufnahmedosis ausgehend von der Grunddosis um einen ganzzahligen Faktor zu reduzieren, so dass durch Addition einer dem ganzzahligen Faktor entsprechenden Anzahl von Durchleuchtungsbildern das geeignete Anzeigebild entsteht.

In einem einfachen Ausführungsbeispiel können mithin zwei Stufen vorgesehen sein, mithin zwei diskrete Aufnahmedosen. In einem solchen Fall kann vorgesehen sein, dass bei Erfüllung eines eine unter eine erste Änderungsschwelle fallende Bildinhaltsveränderung zwischen aufeinanderfolgenden Bildern anzeigenden Reduzierungskriteriums die Aufnahmedosis ausgehend von der Grunddosis reduziert wird und dass bei Erfüllung eines Erhöhungskriteriums, das eine über eine zweite Änderungsschwelle steigende Bildinhaltsveränderung zwischen aufeinanderfolgenden Durchleuchtungsbildern anzeigt, die Aufnahmedosis wieder auf die Grunddosis erhöht wird. Eine entsprechende Anpassung der Bildraten kann in diesem einfachen Ausführungsbeispiel ebenso erfolgen.

In vorteilhaften Ausgestaltungen können jedoch mehr als zwei Stufen, das bedeutet, mehr als zwei diskrete Werte der Aufnahmedosis, verwendet werden. Dies ist insbesondere dann zweckmäßig, wenn die Wahl der Aufnahmedosis ferner abhängig von einem Bildinhaltsveränderungsgradienten über mehr als zwei aufeinanderfolgend aufgenommene, aktuelle Durchleuchtungsbilder erfolgen soll. Dann kann vorgesehen sein, dass bei Überschreitung eines Schwellwerts durch den Bildinhaltsveränderungsgradienten wenigstens eine Stufe übersprungen wird. Zeigt mithin die Geschwindigkeit der Bildinhaltsveränderung an, dass mit einer noch stärkeren Bildinhaltsveränderung in der Zukunft zu rechnen ist, kann gleich in eine Stufe weiterer Reduzierung der Aufnahmedosis umgeschaltet werden, wobei selbstverständlich umgekehrt das Gleiche gelten kann. Jedoch kann auch bei zwei Stufen die Berücksichtigung des Bildinhaltsveränderungsgradienten nützlich sein, beispielsweise, um frühzeitig eine Reduzierung der Aufnahmedosis bzw. eine Erhöhung der Aufnahmedosis auf die Grunddosis vornehmen zu können.

Als Maß für die Bildinhaltsveränderung kann ein insbesondere anwendungsbezogen gewähltes Vergleichsmaß zwischen den zwei aktuellsten Durchleuchtungsbildern gewählt werden. Dabei sind insgesamt unterschiedlichste Vergleichsmaße denkbar, beispielsweise bei Kontrastmitteln durch lokale Analyse des Inhalts von Blutgefäßen, bei nachverfolgten medizinischen Instrumenten deren Verschiebung und dergleichen. Selbstverständlich können auch die Bilddaten der Durchleuchtungsbilder global auswertende Vergleichsmaße herangezogen werden, wenn diese nur hinreichend sensitiv auf die relevanten Veränderungen für die entsprechende Anwendung reagieren.

Konkret kann als Anwendung beispielsweise vorgesehen sein, dass die Durchleuchtungsbilder zur Überwachung eines mit einem Instrument, insbesondere einem Katheter, ausgeführten minimalinvasiven Eingriffs und/oder zur Überwachung einer Kontrastmittelausbreitung innerhalb des Patienten aufgenommen werden. Auch in anderen Anwendungsfällen, bei denen dynamische Veränderungen innerhalb eines Patienten auftreten können, die mittels Durchleuchtungsbildern, insbesondere Fluroskopiebildern, überwacht werden sollen, ist die vorliegende Erfindung selbstverständlich einsetzbar.

Neben dem Verfahren betrifft die Erfindung auch eine Röntgeneinrichtung, die neben wenigstens einer einen Röntgenstrahler und einen Röntgendetektor aufweisenden Aufnahmeanordnung auch eine zur Durchführung des erfindungsgemäßen Verfahrens ausgebildete Steuereinrichtung aufweist. Die Steuereinrichtung kann hierbei auch zur sonstigen Steuerung des Betriebs der Röntgeneinrichtung ausgebildet sein und wenigstens einen Prozessor und ein Speichermittel aufweisen. Insbesondere kann die Steuereinrichtung zur Durchführung des erfindungsgemäßen Verfahrens neben einer Aufnahmeeinheit zur Steuerung des Aufnahmebetriebs der Röntgeneinrichtung auch eine Vergleichseinheit zur Ermittlung einer Bildinhaltsveränderung und eine Anpassungseinheit zur Anpassung der Aufnahmedosis umfassen. Ferner kann in einer bevorzugten Ausgestaltung der Erfindung auch eine Zusammenfassungseinheit zur Zusammenfassung mehrerer aufeinanderfolgend mit reduzierter Aufnahmedosis aufgenommener Durchleuchtungsbilder zu einem Anzeigebild verwendet werden. Die Röntgeneinrichtung umfasst ferner die Anzeigeeinrichtung, beispielsweise einen Monitor, auf dem die Durchleuchtungsbilder, gegebenenfalls zeitweise zusammengefasst als Anzeigebild, ausgegeben werden.

Ein erfindungsgemäßes Computerprogramm ist beispielsweise direkt in einem Speicher einer Steuereinrichtung einer Röntgeneinrichtung ladbar und weist Programmmittel auf, um die Schritte eines erfindungsgemäßen Verfahrens auszuführen, wenn das Computerprogramm in der Steuereinrichtung der Röntgeneinrichtung ausgeführt wird. Ein erfindungsgemäßer elektronisch lesbarer Datenträger umfasst darauf gespeicherte elektronisch lesbare Steuerinformationen, welche zumindest ein erfindungsgemäßes Computerprogramm umfassen und derart ausgestaltet sind, dass sie bei Verwendung des Datenträgers in einer Steuereinrichtung einer Röntgeneinrichtung ein erfindungsgemäßes Verfahren durchführen. Bei dem Datenträger kann es sich insbesondere um einen nichttransienten Datenträger, beispielsweise eine CD-ROM, handeln.

Weitere Vorteile und Einzelheiten der vorliegenden Erfindung ergeben aus den im Folgenden beschriebenen Ausführungsbeispielen sowie anhand der Zeichnung. Dabei zeigen:
- Fig. 1: einen generellen Ablaufplan eines Ausführungsbeispiels des erfindungsgemäßen Verfahrens,
- Fig. 2: einen möglichen Verlauf verschiedener Größen während einer Fluroskopie-Überwachung, und
- Fig. 3: eine erfindungsgemäße Röntgeneinrichtung.

Im Folgenden sollen Ausführungsbeispiele des erfindungsgemäßen Verfahrens anhand einer fluroskopischen Überwachung bei einer Anwendung an einem Patienten, beispielsweise einem minimalinvasiven Eingriff mit einem medizinischen Instrument, dargestellt werden. Hierbei wird eine Röntgeneinrichtung verwendet, die eine Aufnahmeanordnung aufweist, beispielsweise an einem C-Bogen, über die mit einer bereits geringen Grunddosis als Aufnahmedosis Durchleuchtungsbilder eines Patienten aufgenommen werden können, die hinreichend deutlich sich dynamisch verändernde, zu überwachende Merkmale im Aufnahmebereich des Patienten zeigen. Es wird also eine Bildserie von Durchleuchtungsbildern, hier Fluoroskopiebildern, aufgenommen. Um die Dosisbelastung des Patienten zu reduzieren, wird bei unveränderter Aufnahmerate die Aufnahmedosis ausgehend von der Grunddosis reduziert und es werden konsequenterweise mehrere Durchleuchtungsbilder zu einem Anzeigebild zusammengefasst, um die der Grunddosis entsprechende Qualität bei der Anzeige wieder zu erhalten, wobei die Bildrate an einer entsprechenden Anzeigeeinrichtung reduziert wird.

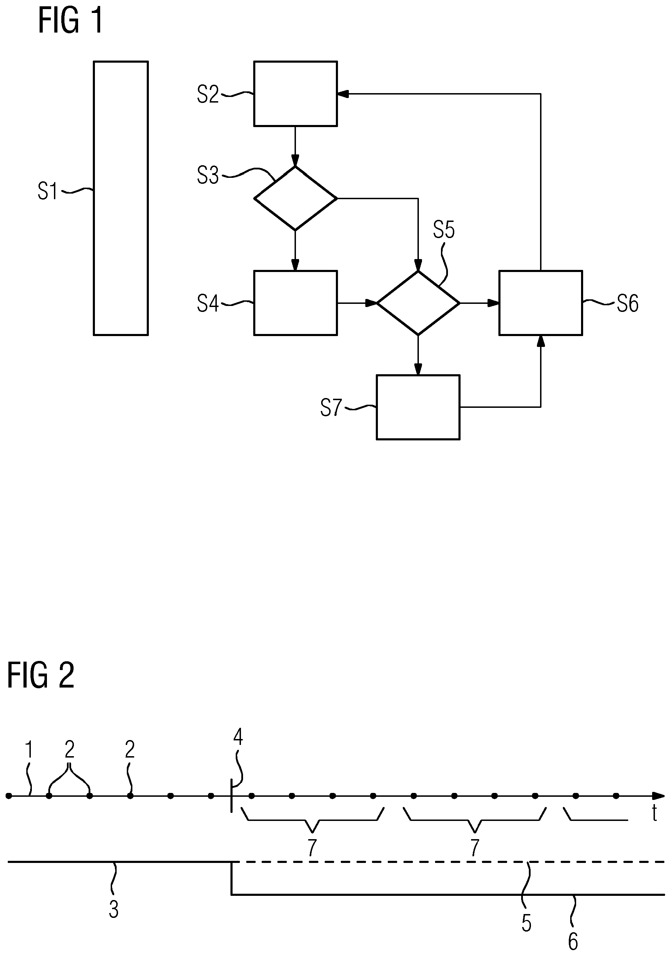

Fig. 1 zeigt einen generellen Ablaufplan eines Ausführungsbeispiels des erfindungsgemäßen Verfahrens. Dabei deutet der Schritt S1 die laufende Überwachung durch kontinuierliche Aufnahme von Durchleuchtungsbildern des Aufnahmebereichs mit der Röntgeneinrichtung an, wobei die Aufnahmerate konstant gehalten wird. Immer dann, wenn ein neues Durchleuchtungsbild vorliegt, wird in einem Schritt S2 durch Vergleich der beiden aktuellsten Durchleuchtungsbilder ein Maß für eine Bildinhaltsveränderung, insbesondere anwendungsspezifisch, bestimmt, vorliegend, indem ein anwendungsspezifisches Vergleichsmaß auf das Paar von zwei aktuellsten Durchleuchtungsbildern angewendet wird.

Diese so ermittelte Bildinhaltsveränderung wird in einem Schritt S3 durch wenigstens ein Auswertungskriterium ausgewertet, um festzustellen, ob eine Anpassung der Aufnahmedosis, insbesondere eine Reduzierung ausgehend vom Grundwert, auf Grund nur langsam oder überhaupt nicht auftretender Veränderungen, sinnvoll ist, bzw. ob eine Erhöhung der Aufnahmedosis auf Grund aufgetretener plötzlicher/schneller/stärkerer Bildinhaltsveränderungen sinnvoll ist.

Ist eine Veränderung der Aufnahmedosis sinnvoll, wird diese im Schritt S4 durchgeführt. Dabei sind in den vorliegenden Ausführungsbeispielen wenigstens zwei zu verwendende Aufnahmedosen, bevorzugt mehr Aufnahmedosen, vorgesehen, wobei die Aufnahmedosen, auf die reduziert werden kann, einem ganzzahligen Anteil der Grunddosis entsprechen. Insbesondere sind verschiedenen Intervallen von Bildinhaltsveränderungen bestimmte, diskrete Werte von Aufnahmedosen zugeordnet. Dabei sei in diesem Zusammenhang angemerkt, dass die Intervalle selbstverständlich wiederum abhängig von der aktuell eingestellten Aufnahmedosis gewählt werden können, da im Schritt S2 ja immer die beiden aktuellsten Durchleuchtungsbilder miteinander verglichen werden, welche je nach Aufnahmedosis unterschiedlich aussehen können und mithin auch unterschiedliche Wertebereiche eines Vergleichsmaßes zur Folge haben können. Insbesondere sind also für jede Aufnahmedosis Bildinhaltsveränderungsintervalle mit zugeordneten Aufnahmedosen vorgesehen, so dass die Zuordnung zu einem bestimmten dieser Intervalle ein Umschalten gemäß dem Schritt S4 zur Folge hat.

In einem Schritt S5 wird überprüft, ob gerade mit einer gegenüber der Grunddosis, die die maximal für die Fluoroskope erlaubte Aufnahmedosis darstellt, reduzierten Aufnahmedosis gearbeitet wird. Ist dies nicht der Fall, wird in einem Schritt S6 das aktuelle Durchleuchtungsbild auf der Anzeigeeinrichtung dargestellt, so dass insbesondere die Bildrate an der Anzeigeeinrichtung der Aufnahmerate entspricht.

Wird jedoch mit verringerter Aufnahmedosis gearbeitet, werden in einem Schritt S7 mehrere Durchleuchtungsbilder zu einem Anzeigebild zusammengefast, dass in seinem Eindruck einem Durchleuchtungsbild, dass mit der Grunddosis aufgenommen wurde, entspricht. Wurde beispielsweise die Aufnahmedosis, wie oben erläutert, um einen Faktor k verringern, werden jeweils disjunkte Gruppen von k Durchleuchtungsbildern zu einem anderen Anzeigebild zusammengefast, das dann im Schritt S6 dargestellt wird.

Es versteht sich, dass während des Sammelns zusammenzufassende Durchleuchtungsbilder im Schritt S2 jeweils für die zwei aktuellsten Durchleuchtungsbilder jedoch kontinuierlich weiter überwacht wird, ob eine relevante Bildinhaltsveränderung vorliegt. Das bedeutet, auch wenn die Bildrate für die Darstellung aus dem Grund, dass Anzeigebilder nur alle k Durchleuchtungsbilder erzeugt werden, bei reduzierter Aufnahmedosis ebenso reduziert ist, dennoch äußerst schnell auf auftretende plötzliche, starke Bildinhaltsveränderungen reagiert werden kann.

Dieses Vorgehen sei durch Fig. 2 näher erläutert. Gezeigt ist zunächst ein Zeitstrahl 1 mit regelmäßig aufeinanderfolgenden Aufnahmezeitpunkten 2 für Durchleuchtungsbilder. Unterhalb des Zeitstrahls 1 ist ein Verlauf 3 der verwendeten Aufnahmedosis gezeigt. Wie ersichtlich ist, wurde zu einem Zeitpunkt 4 im Schritt S3 festgestellt, dass beispielsweise ein Reduzierungskriterium aufgrund äußerst geringer Bildinhaltsveränderungen erfüllt ist, so dass die Aufnahmedosis gemäß dem Schritt S4 von der Grunddosis 5 auf einen niedrigeren Wert 6, hier ein Viertel der Grunddosis 5, reduziert wurde. Ab dem Zeitpunkt 4 werden mithin gemäß dem Schritt S7 immer vier Durchleuchtungsbilder, vgl. Klammer 7, zusammengefasst, vorliegend addiert, um Anzeigebilder zu erzeugen, so dass sich die Bildrate bei der Darstellung im Schritt S6 ebenso viertelt. Dabei ist die Reduzierung um den Faktor Vier hier als reines Beispiel zu verstehen.

Wie bereits erwähnt, müssen dabei nicht zwangsläufig nur zwei derartige Stufen vorgesehen sein, sondern sind auch drei oder mehr Stufen denkbar. Geraden an einem solchen Kontext, aber auch bei der Verwendung von nur zwei Stufen, sieht eine zweckmäßige, optionale Weiterbildung der Schritte S2 und S3 vor, dass im Schritt S2 zusätzlich auch ein Gradient der Bildinhaltsveränderung über mehr als zwei aufeinanderfolgend aufgenommene, aktuelle Durchleuchtungsbilder ermittelt wird. Beispielsweise können hier jeweils die letzten zwei bis zehn aufgenommenen Durchleuchtungsbildpaare betrachtet werden. Dann kann beurteilt werden, ob es sich bei einer aufgetretenen Bildinhaltsveränderung um einen beispielsweise nur ein Durchleuchtungsbildpaar betreffenden "Ausreißer" gehandelt hat, so dass mithin eine Ausreißerdetektion durchgeführt werden kann, wobei bei einem detektierten Ausreißer, insbesondere einem einzelnen Ereignis, kein zwangsläufiges Umschalten im Schritt S3 erfolgen muss. Bevorzugt ist es zusätzlich oder alternativ jedoch auch möglich, den Bildinhaltsveränderungsgradienten als einen Trend zu begreifen, beispielsweise im Sinne der Beschreibung eines medizinischen Instruments, dass sich in Bewegung gesetzt hat, sodass, insbesondere anwendungsbezogen, auch abgeschätzt werden kann, auf welches Niveau von Bildinhaltsveränderungen die aktuellen dynamischen Vorgänge hinauslaufen. Abhängig hiervon kann beispielsweise eine Stufe der Aufnahmedosis übersprungen werden und/oder es kann gegebenenfalls im Schritt S3 auch ein früheres, vorausschauendes Umschalten auf eine andere, insbesondere auch höhere, Aufnahmedosis erfolgen.

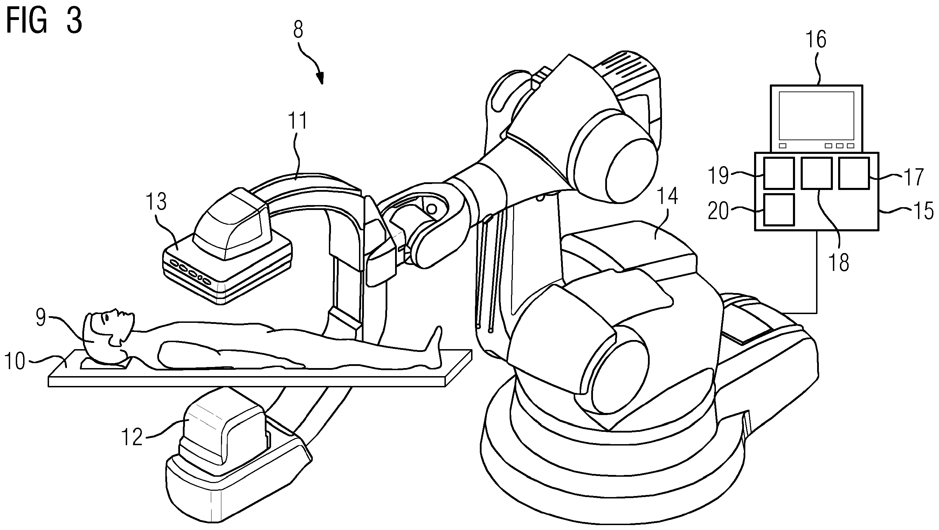

Fig. 3 zeigt schließlich ein Ausführungsbeispiel einer erfindungsgemäßen Röntgeneinrichtung 8, wie sie beispielsweise an einem Arbeitsplatz für eine minimalinvasive medizinische Intervention eingesetzt werden kann, bei der ein Patient 9 auf einer Patientenliege 10 platziert wird. Die Röntgeneinrichtung 8 weist einen C-Bogen 11 auf, an dem sich gegenüberliegend ein Röntgenstrahler 12 und ein Röntgendetektor 13 als Aufnahmeanordnung angeordnet sind. Der C-Bogen 11 ist hierbei vorliegend an einem Roboterarm 14 als Stativ gekoppelt, wobei auch andere Ausgestaltungen selbstverständlich denkbar sind.

Der Betrieb der Röntgeneinrichtung 8 wird über eine Steuereinrichtung 15 gesteuert, der vorliegend auch eine Anzeigeeinrichtung 16, beispielsweise ein Überwachungsmonitor, zugeordnet ist. Die Steuereinrichtung 15 ist zur Durchführung des erfindungsgemäßen Verfahrens ausgebildet, wozu sie vorliegend neben einer allgemein den Aufnahmebetrieb gemäß gewählter Aufnahmeparameter steuernden Aufnahmeeinheit 17 auch eine Vergleichseinheit 18 zur Ermittlung der Bildinhaltsveränderung (gegebenenfalls auch des Bildinhaltsveränderungsgradienten) und eine Anpassungseinheit 19 zur Anpassung der Aufnahmedosis aufweist. Ferner ist eine Kommunikationseinheit 20 zur Ermittlung eines Anzeigebilds durch Kombination mehrerer Durchleuchtungsbilder gezeigt. Selbstverständlich sind auch weitere Subeinheiten grundsätzlich denkbar.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

### Bezugszeichenliste

- 1: Zeitstrahl
- 2: Aufnahmezeitpunkt
- 3: Verlauf
- 4: Zeitpunkt
- 5: Grunddosis
- 6: Wert
- 7: Klammer
- 8: Röntgeneinrichtung
- 9: Patient
- 10: Patientenliege
- 11: C-Bogen
- 12: Röntgenstrahler
- 13: Röntgendetektor
- 14: Roboterarm
- 15: Steuereinrichtung
- 16: Anzeigeeinrichtung
- 17: Aufnahmeeinheit
- 18: Vergleichseinheit
- 19: Anpassungseinheit
- 20: Kommunikationseinheit

- S1 - S7: Schritt

## Patentansprüche

1. Verfahren zum Betrieb einer Röntgeneinrichtung (8) zur Aufnahme einer Bildserie zur insbesondere fluoroskopischen Überwachung eines Aufnahmebereichs eines Patienten (9), wobei Durchleuchtungsbilder mit einer Aufnahmerate und einer Aufnahmedosis erfasst und mit einer Bildrate einem Benutzer an einer Anzeigeeinrichtung (16) dargestellt werden, wobei die Aufnahmedosis pro Durchleuchtungsbild in Abhängigkeit einer Bildinhaltsveränderung zwischen aktuellen, aufeinanderfolgenden Durchleuchtungsbildern automatisch gewählt wird, wobei als Maß für die Bildinhaltsveränderung ein Vergleichsmaß zwischen den zwei aktuellsten Durchleuchtungsbildern gewählt wird, und wobei bei einer Reduzierung der Aufnahmedosis aus jeweils mehreren aufeinanderfolgend aufgenommenen Durchleuchtungsbildern ein Anzeigebild, das einer maximal verwendbaren Grunddosis (5) entspricht, ermittelt und an der Anzeigeeinrichtung (16) ausgegeben wird.

2. Verfahren nach Anspruch 1, **dadurch gekennzeichnet, dass** bei einer Ermittlung von Anzeigebildern die Bildrate reduziert wird, insbesondere um einen Faktor entsprechend der Anzahl der zusammengefassten Durchleuchtungsbilder bei disjunkten Gruppen zusammenzufassender Durchleuchtungsbilder.

3. Verfahren nach einem der vorangehenden Ansprüche, **dadurch gekennzeichnet, dass** die Wahl der Aufnahmedosis ferner abhängig von einem Gradienten der Bildinhaltsveränderung über mehr als zwei aufeinander folgend aufgenommene, aktuelle Durchleuchtungsbilder erfolgt.

4. Verfahren nach einem der vorangehenden Ansprüche, **dadurch gekennzeichnet, dass** die Veränderung der Aufnahmedosis in diskreten Stufen abhängig von der Überschreitung oder Unterschreitung von wenigstens einer Änderungsschwelle durch die Bildinhaltsveränderung erfolgt.

5. Verfahren nach Anspruch 4, **dadurch gekennzeichnet, dass** bei mehr als zwei Stufen bei Überschreitung wenigstens eines Schwellwerts durch einen gemäß Anspruch 3 ebenso berücksichtigten Bildinhaltsveränderungsgradienten wenigstens eine Stufe übersprungen wird.

6. Verfahren nach Anspruch 4 oder 5, **dadurch gekennzeichnet, dass** die Stufen durch Reduzierung der Aufnahmedosis ausgehend von der Grunddosis um einen ganzzahligen Faktor definiert werden, so dass durch Addition einer dem ganzzahligen Faktor entsprechenden Anzahl von Durchleuchtungsbildern ein Anzeigebild, das der Grunddosis entspricht, entsteht.

7. Verfahren nach einem der vorangehenden Ansprüche, **dadurch gekennzeichnet, dass** das Maß für die Bildinhaltsveränderung anwendungsbezogen gewählt wird.

8. Verfahren nach einem der vorangehenden Ansprüche, **dadurch gekennzeichnet, dass** die Durchleuchtungsbilder zur Überwachung eines mit einem Instrument, insbesondere einem Katheter, ausgeführten minimalinvasiven Eingriffs und/oder einer Kontrastmittelausbreitung innerhalb des Patienten (9) aufgenommen werden.

9. Röntgeneinrichtung (8) mit einer zur Durchführung eines Verfahrens nach einem der vorangehenden Ansprüche ausgebildeten Steuereinrichtung (15) und einer Anzeigeeinrichtung (16).

10. Computerprogramm, welches die Schritte eines Verfahrens nach einem der Ansprüche 1 bis 8 durchführt, wenn es auf einer Steuereinrichtung (15) einer Röntgeneinrichtung (8) durchgeführt wird.

11. Elektronisch lesbarer Datenträger, auf dem ein Computerprogramm nach Anspruch 10 gespeichert ist.

## Claims

1. Method for operating an x-ray device (8) for recording a series of images for in particular fluoroscopic monitoring of a recording region of a patient (9), wherein fluoroscopy images are acquired with a recording rate and a recording dose and are displayed to a user on a display device (16) with a frame rate, wherein the recording dose is selected automatically per fluoroscopy image as a function of a change in image contents between the current consecutive fluoroscopy images, wherein a comparison measure between the two most up to date fluoroscopy images is selected as a measure of the change in image contents, and wherein with a reduction in the recording dose a display image, which corresponds to a maximally usable base dose (5), is determined from in each case a number of fluoroscopy images recorded consecutively and output to the display device (16).

2. Method according to claim 1, **characterised in that** the frame rate is reduced when display images are determined, in particular by a factor according to the number of combined fluoroscopy images with disjointed groups of fluoroscopy images to be combined.

3. Method according to one of the preceding claims, **characterised in that** the recording dose is also selected as a function of a gradient of the change in image contents by way of more than two current fluoroscopy images recorded consecutively.

4. Method according to one of the preceding claims, **characterised in that** the change in recording dose takes place in discrete stages as a function of at least one rearrangement threshold being exceeded or not met by the change in image contents.

5. Method according to claim 4, **characterised in that** with more than two stages, at least one stage is skipped if at least one threshold value is exceeded by a change in image content gradients likewise taken into account in accordance with claim 3.

6. Method according to claim 4 or 5, **characterised in that** the stages are defined by reducing the recording dose by an integral factor starting from the base dose, so that by adding a number of fluoroscopy images which corresponds to the integral factor, a display image which corresponds to the base dose appears.

7. Method according to one of the preceding claims, **characterised in that** the measure of the change in image contents is selected depending on the application.

8. Method according to one of the preceding claims, **characterised in that** the fluoroscopy images for monitoring a minimally invasive intervention carried out with an instrument, in particular a catheter, and/or a contrast agent propagation within the patient (9) are recorded.

9. X-ray device (8) with a control device (15) embodied to carry out a method according to one of the preceding claims and a display device (16).

10. Computer program, which performs the steps of a method according to one of claims 1 to 8, when it is executed on a control device (15) of an x-ray device (8).

11. Electronically readable data carrier, on which is stored a computer program according to claim 10.

## Revendications

1. Procédé pour faire fonctionner un dispositif (8) à rayons x d'enregistrement d'une série d'images, notamment pour le contrôle fluoroscopique d'une région à enregistrer d'un patient (9), dans lequel on prend des radiographies à un taux d'enregistrement et à une dose d'enregistrement et on les représente avec un taux d'image à un utilisateur sur un dispositif (16) d'affichage, dans lequel on choisit automatiquement la dose d'enregistrement par radiographie, en fonction d'une variation du contenu de l'image entre des radiographies récentes successives, dans lequel, comme mesure de la variation du contenu d'image, on choisit une mesure de comparaison entre les deux radiographies les plus récentes, et dans lequel, lors d'une réduction de la dose d'enregistrement, on détermine, à partir de, respectivement, plusieurs radiographies enregistrées successivement, une image d'affichage, qui correspond à une dose (5) de base pouvant être utilisée au maximum et on la donne au dispositif (16) d'affichage.

2. Procédé suivant la revendication 1, **caractérisé en ce que**, lors d'une détermination d'images d'affichage, on réduit le taux d'image, notamment d'un facteur correspondant au nombre de radiographies rassemblées pour des groupes disjoints de radiographies à rassembler.

3. Procédé suivant l'une des revendications précédentes, **caractérisé en ce que** le choix de la dose d'enregistrement s'effectue, en outre, en fonction d'un gradient de la variation du contenu d'image sur plus de deux radiographies récentes enregistrées successivement.

4. Procédé suivant l'une des revendications précédentes, **caractérisé en ce que** la variation de la dose d'enregistrement s'effectue en pas discret en fonction du fait que la variation du contenu d'images passe au-dessus ou en dessous d'au moins un seuil de variation.

5. Procédé suivant la revendication 4, **caractérisé en ce que**, pour plus de deux pas, lors d'un dépassement d'au moins une valeur de seuil par un gradient de variation du contenu d'images pris en compte également suivant la revendication 3, on saute au moins un pas.

6. Procédé suivant la revendication 4 ou 5, **caractérisé en ce que** l'on définit les pas par réduction de la dose d'enregistrement à partir de la dose de base d'un facteur en nombre entier, de manière à créer, par addition d'un nombre de radiographies correspondant au facteur en nombre entier, une image d'affichage, qui correspond à la dose de base.

7. Procédé suivant l'une des revendications précédentes, **caractérisé en ce que** l'on choisit la mesure pour la variation du contenu d'image en la rapportant à l'application.

8. Procédé suivant l'une des revendications précédentes, **caractérisé en ce que** l'on enregistre les radiographies pour le contrôle d'une intervention invasive au minimum effectuée par un instrument, notamment un cathéter et/ou pour la diffusion d'un agent de contraste.

9. Dispositif (8) à rayons X ayant un dispositif (15) de commande constitué pour effectuer un procédé suivant l'une des revendications précédentes et un dispositif (16) d'affichage.

10. Programme d'ordinateur, qui effectue les stades d'un procédé suivant l'une des revendications 1 à 8, lorsqu'il est effectué sur un dispositif (15) de commande d'un dispositif (8) à rayons X.

11. Support de données déchiffrables électroniquement, sur lequel est mémorisé un programme d'ordinateur suivant la revendication 10.
